# EUROPÄISCHE PATENTANMELDUNG

(11) **EP 2 397 117 A1**
(43) Veröffentlichungstag der Anmeldung: **21.12.2011**
(21) Anmeldenummer: 10166154.4
(22) Anmeldetag: 16.06.2010
(51) Int. Cl.: A61H 21/00, A61H 23/02, A61F 13/20

(54) **Verfahren zum Umhüllen eines Körpers mit Fasermaterial**

(71) Anmelder: Ruggli Projects AG, 6332 Hagendorn (CH)
(72) Erfinder: Kaiser, Rudolf, 79787 Lauchringen (DE)
(74) Vertreter: AMMANN PATENTANWÄLTE AG BERN

(57) **Zusammenfassung**

Die Erfindung löst das Problem, einen Körper, beispielsweise einen in einer Kapsel enthaltenen Vibrationsgenerator, mit einem Fasermaterial zu umhüllen, um beispielsweise einen Tampon (2) zu bilden. Die Lösung besteht darin, dass das Fasermaterial um den Körper herum gewickelt wird.

## Beschreibung

Die Erfindung betrifft ein Verfahren zum Umhüllen eines Körpers mit Fasermaterial.

Aus den Dokumenten US5782779,US6183428 und US2007/0260210 sind Tamponanordnungen mit einem im Tamponkörper eingebetteten Vibrationselement bekannt. Solche Tamponanordnungen werden gegen Menstruationsbeschwerden eingesetzt. Was die Einbettung des Vibrationselements in den Tamponkörper anbelangt, kann lediglich dem Dokument US2007/0260210 entnommen werden, dass das Vibrationselement in einer Kapsel enthalten ist, an deren Aussenseite Rückhalteelemente angeordnet sind, um zu verhindern, dass die Kapsel insbesondere beim Zurückziehen der Anordnung aus der Vagina aus dem Tamponkörper herausgerissen wird. Der Tamponkörper ist in diesem Dokument als Hohlkörper beschrieben, in den die Kapsel offenbar hineingesteckt wird. Einerseits ist die Herstellung eines Hohlkörpers aus Fasermaterial nicht einfach, andererseits bedeutet das anschliessende Zusammenfügen mit der Kapsel einen zusätzlichen Aufwand.

Ausgehend von diesem Stand der Technik liegt der Erfindung die Aufgabe zu Grunde, ein Verfahren vorzuschlagen, das die genannten Nachteile nicht aufweist und insbesondere einfach, schnell und kostengünstig durchführbar ist.

Diese Aufgabe wird erfindungsgemäss dadurch gelöst, dass das Fasermaterial um den Körper herum gewickelt wird.

Diese erfindungsgemässe Lösung hat den Vorteil, dass durch das Wickeln des Fasermaterials um den Körper Arbeitsgänge für die separate Herstellung eines Hohlkörpers aus Fasermaterial entfallen.

Nach einer Ausführungsart des erfindungsgemässen Verfahrens ist das Fasermaterial als Flächengebilde ausgebildet. Ein Flächengebilde eignet sich gut zur Bildung einer Umhüllung.

Bevorzugt ist das das Fasermaterial als Band ausgebildet. Ein Band ist besonders gut zur Bildung einer Umhüllung geeignet, die schliesslich einen saugfähigen Körper bilden soll.

Nach einer anderen Ausführungsart ist der Körper eine vorzugsweise längliche Kapsel mit einem Inhalt. Als Inhalt sind diagnostische oder therapeutische Einrichtungen inklusive Einrichtungen zur Abgabe von Wirkstoffen denkbar.

Nach einer weiteren Ausführungsart ist vorgesehen, dass der Inhalt der Kapsel einen Energieverbraucher umfasst. Energieverbraucher können beispielsweise elektrisch, pneumatisch oder hydraulisch betriebene Vorrichtungen sein.

Gemäss einer weiteren Ausführungsart ist der Energieverbraucher ein Vibrationsgenerator. Eine derartige Anordnung kann beispielsweise, wie eingangs erwähnt, zur Beeinflussung der Menstruation verwendet werden.

Eine andere Ausführungsart sieht vor, dass der Körper mit einem flexiblen Zugmittel verbunden ist, das vorzugsweise mindestens einen Leiter für Energie enthält. Das Zugmittel kann zur Zurückziehen der Anordnung aus einer Körperöffnung dienen und der Leiter zur Versorgung eines Verbrauchers mit Energie.

Nach einer weiteren Ausführungsart wird der Körper in einem Haltewerkzeug gehalten und das Haltewerkzeug mit dem darin gehaltenen Körper und das Fasermaterial werden relativ zueinander rotiert, um einen Wickel zu bilden. Auf diese Weise werden relativ einfach und rasch mehrere Lagen aus dem Fasermaterial um den Körper aufgebaut.

Gemäss einer weiteren Ausführungsart ist das Haltewerkzeug gabelförmig und weist mindestens zwei Gabelschenkel auf. Zwischen diesen zwei Schenkeln kann der Körper gehalten werden.

Nach einer weiteren Ausführungsart wird das Fasermaterial vor dem Rotieren zwischen den Körper und mindestens einen der Gabelschenkel gebracht. Dadurch ist das Fasermaterial relativ zum Körper festgehalten und kann aufgewickelt werden.

Eine andere Ausführungsart sieht vor, dass das Fasermaterial vor dem Rotieren zwischen den Körper und mindestens zwei der Gabelschenkel gebracht wird. So wird der Körper bereits vor dem Rotieren auf einem Teil seines Umfangs von Fasermaterial umschlungen.

Gemäss einer weiteren Ausführungsart wird das Fasermaterial in einer Schleife um den Körper gelegt und das flexible Zugmittel wird über die Schleife parallel zum Körper geführt. Durch die so entstandene Umlenkung des flexiblen Zugmittels resultiert eine ausgezeichnete Ausreisssicherheit des Zugmittels aus dem Körper und später auch des Körpers aus dem Wickel.

Nach einer weiteren Ausführungsart wird der Körper in das Haltewerkzeug eingesetzt, das Fasermaterial wird neben dem Haltewerkzeug axial neben dem Körper im Bereich des Zugmittels platziert und der Körper wird um eine senkrecht zu seiner Längsachse verlaufende Achse gewendet, derart, dass der Körper das Fasermaterial unter Bildung der Schleife in das Haltewerkzeug zieht. Damit wird in einfacher Weise die vorangehend erwähnte Umlenkung des Zugmittels erreicht.

Nach einer weiteren Ausführungsart wird nach dem Rotieren der entstandene Wickel axial vom Haltewerkzeug abgestreift und in ein Formwerkzeug gebracht. Durch dieses Formwerkzeug wird dem Wickel die endgültige Form, beispielsweise die Form eines Tampons verliehen. Vorzugsweise hat dabei das Formwerkzeug einen Innendurchmesser, der kleiner ist als der Aussendurchmesser des Wickels. Dadurch wird das Fasermaterial im Formwerkzeug komprimiert.

Nach einer zusätzlichen Ausführungsart wird dem Wickel mit dem Formwerkzeug eine gerundete Spitze gegeben. Diese Spitze erleichtert das Einführen der Anordnung, beispielsweise in eine Körperöffnung.

Schliesslich ist nach einer weiteren Ausführungsart vorgesehen, dass das Formwerkzeug beheizt wird. Dadurch wird insbesondere erreicht, dass der Wickel den verformten Zustand beibehält.

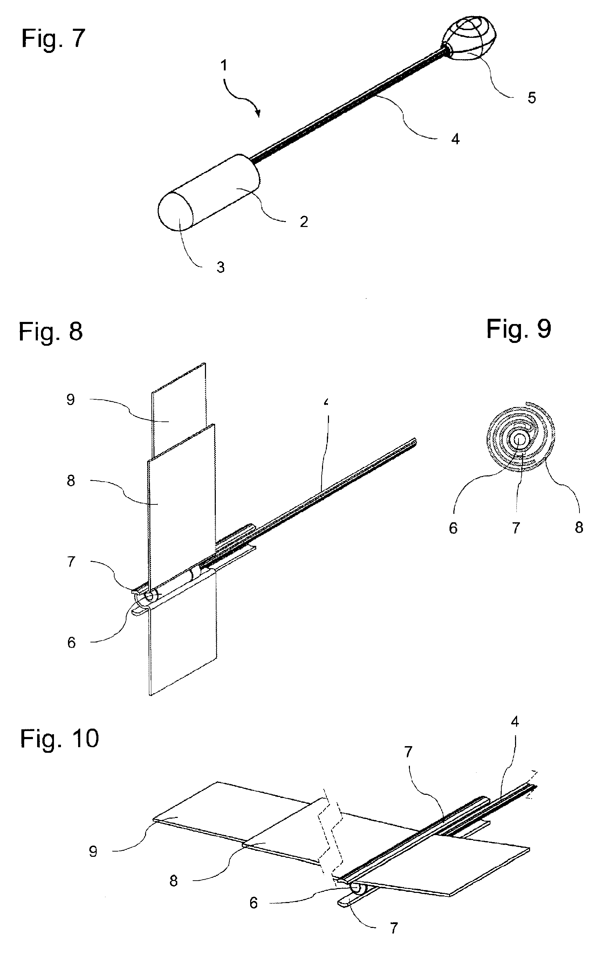

Ausführungsarten des erfindungsgemässen Verfahrens werden nun am Beispiel der Herstellung einer Tamponanordnung unter Bezugnahme auf die angefügten Zeichnungen erläutert: Es zeigt
- Figur 1: eine perspektivische Ansicht einer vormontierten Anordnung mit einer Kapsel und eines Haltewerkzeugs in der Ausgangslage vor dem Zusammenfügen mit Fasermaterial,
- Figur 2: die Anordnung im Haltewerkzeug und ein darum gelegtes Faserband,
- Figur 3: einen Wendevorgang der Kapsel,
- Figur 4: eine Draufsicht auf die Anordnung und das Haltewerkzeug vor dem Wickeln,
- Figur 5: eine perspektivische Ansicht der Anordnung und des Haltewerkzeugs nach dem Wickeln,
- Figur 6: die Anordnung mit weiteren Werkzeugen,
- Figur 7: eine fertige Tamponanordnung,
- Figur 8: eine alternative Anordnung der Komponenten vor dem Wickeln,
- Figur 9: ausgehend von Figur 8 eine Frontansicht der Komponenten nach dem Wickeln,
- Figur 10: eine weitere alternative Anordnung der Komponenten vor dem Wickeln,
- Figuren 11 und 12: eine weitere alternative Anordnung der Komponenten vor dem Wickeln und
- Figur 13: ausgehend von den Figuren 11 und 12 eine Frontansicht der Komponenten nach dem Wickeln.
Ausführungsbeispiele des erfindungsgemässen Verfahrens werden nachstehend an Hand der Herstellung einer Tamponanordnung 1 (siehe Figur 7) mit einem Vibrationsgenerator beschrieben. Die Erfindung ist aber nicht auf die Herstellung derartiger Tamponanordnungen beschränkt. Vielmehr ist die Erfindung auf die Herstellung beliebiger Körper anwendbar, die mit Fasermaterial umhüllt werden. Ferner könnte der Körper beispielsweise ein Heizelement oder Sensoren enthalten oder als Kapsel mit einem Medikament ausgebildet sein.

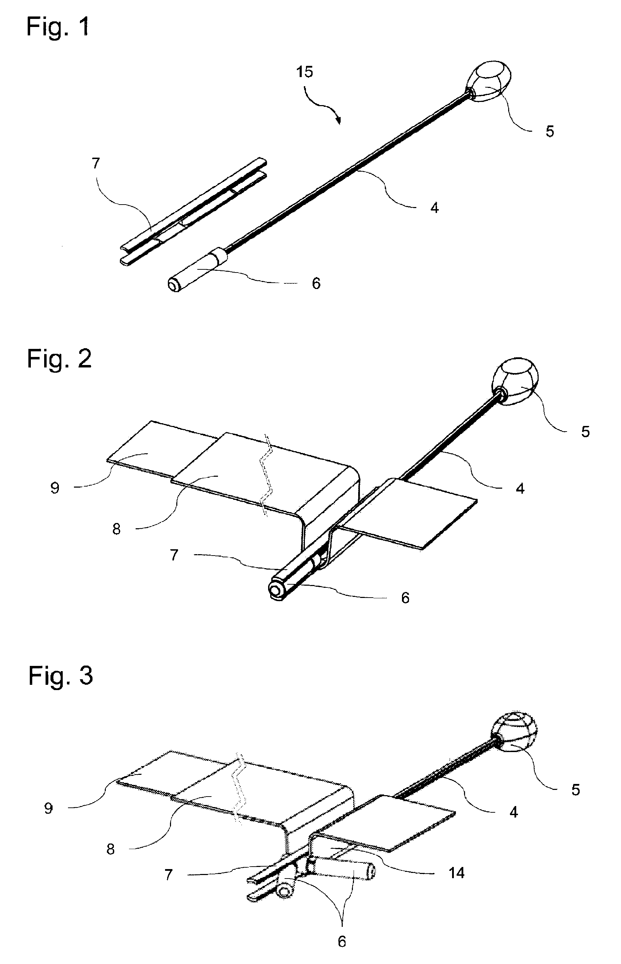

Figur 1 zeigt eine vormontierte Anordnung 15, bestehend aus einer einen Vibrationsgenerator enthaltenden Kapsel 6, einem zur Stromleitung und als Rückzugsmittel dienenden Kabel 4 und einer Energieversorgungs- und Steuereinheit 5. Es ist das Ziel des nachfolgend beschriebenen Verfahrens, einen Tampon 2 (Figur 7) herzustellen, der die Kapsel 6 mindestens teilweise umgibt. Mit 7 ist ein gabelförmiges Haltewerkzeug mit zwei Gabelschenkeln bezeichnet, das dazu bestimmt ist, die Kapsel 6 aufzunehmen und zu halten. Die Kapsel 6 wird vorzugsweise ausgehend von der in Figur 1 dargestellten Position seitwärts zwischen die Gabelschenkel des Haltewerkzeuges 7 geschoben.

Figur 2 zeigt das Haltewerkzeug 7 mit eingelegter Kapsel 6. Ein Faserband 8 ist am kabelseitigen Ende der Kapsel in einer Schleife um das Haltewerkzeug 7 herum gelegt, derart, dass die Kapsel 6 nicht durch das Faserband bedeckt wird. Ein Vliesband 9 ist mit dem Faserband 8 verbunden und dafür vorgesehen, am Schluss des Verfahrens die Oberfläche des fertigen Tampons mindestens teilweise zu bedecken.

In Figur 3 ist gezeigt, wie die Kapsel im Haltewerkzeug 7 gewendet wird. Dabei sind zwei Zwischenpositionen der Kapsel 6 mit gestrichelten Linien dargestellt. Wird die Kapsel aus der in Figur 3 dargestellten Position im Gegenuhrzeigersinn weiter gedreht, kommt sie im mit der Bezugszahl 14 bezeichneten Bereich mit der Aussenoberfläche der Schleife in Kontakt und zieht beim weiter Bewegen das Faserband 8 zwischen die Gabelschenkel des Haltewerkzeugs 7. Nachdem der Wendevorgang beendet ist, liegt die Kapsel gegenüber Figur 2 um 180 Grad gedreht koaxial im Haltewerkzeug 7 und das Faserband 8 ist unter Bildung einer Schleife zwischen der Kapsel 6 und den beiden Gabelschenkeln des Haltewerkzeugs 7 eingeklemmt, während das Kabel 4 ausserhalb dieser Schleife parallel zur Kapsel 6 verläuft. Durch diesen Verfahrensschritt ergeben sich zwei wesentliche Vorteile. Erstens ist das Kabel 4 am Ende der Kapsel 6 umgelenkt, wodurch die Sicherheit gegen das Ausreissen des Kabels 4 aus der Kapsel 6 erhöht wird. Zweitens verläuft das Kabel 4 vom Ende der Kapsel um den Längsrand des Faserbands 8 herum, wodurch schlussendlich die Sicherheit gegen das Ausreissen der Kapsel 6 aus dem Tampon erhöht wird.

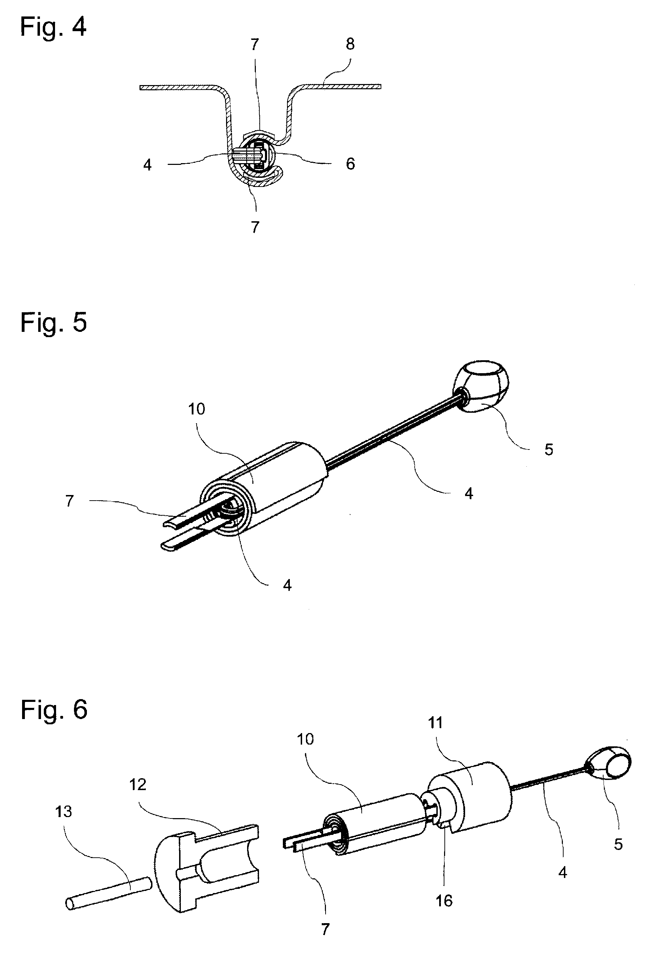

Wenn am Ende des Verfahrensschrittes gemäss Figur 3 die Kapsel 6 koaxial im Haltewerkzeug 7 sitzt, wird dieses bezogen auf die Figur im Gegenuhrzeigersinn rotiert, und zwar mitsamt dem Kabel 4 und der Energieversorgungs- und Steuereinheit 5. Währenddessen wird das Faserband 8 geführt, so dass es um das Haltewerkzeug 7 und die darin gehaltene Kapsel 6 herum gewickelt wird. Figur 4 zeigt eine Draufsicht auf die Anordnung 1 und das Haltewerkzeug 7 ausgehend von der Darstellung in Figur 3, nach abgeschlossenem Wendevorgang. Die Kapsel 6 ist vom Faserband 8 umschlungen und liegt zwischen den Schenkeln des Haltewerkzeugs 7. Durch Drehen des Haltewerkzeugs 7 um seine Längsachse werden nun die beiden freien Enden des Faserbandes 8 aussen um die beiden Gabelschenkel herum gewickelt. Die Figur zeigt auch, dass das Kabel 4 aussen über die erste Schicht des Faserbandes 8 geführt ist und in der Folge von weiteren Lagen des Faserbandes 8 umwickelt wird. Es ist auch möglich, das Faserband 8 durch geeignete Mittel um das Haltewerkzeug 7 mit der darin gehaltenen Kapsel 6 herum zu wickeln, während das Haltewerkzeug 7 stillsteht. Auch eine kombinierte, gegenläufige Rotationsbewegung des Haltewerkzeugs 7 und des Faserbandes 8 ist prinzipiell möglich.

Figur 5 zeigt den durch die beschriebene Rotationsbewegung entstandenen Wickel 10 und das Haltewerkzeug 7. Auch hier ist sichtbar, dass das Kabel 4 ausgehend vom Ende der Kapsel 6 um diese herum gelenkt ist, derart dass das Faserband 8 in diesem Bereich zwischen der Kapsel 6 und dem Kabel 4 verläuft.

Figur 6 zeigt den Wickel 10 vor dem nächsten Verfahrensschritt, welcher das Abstreifen des Wickels 10 vom Haltewerkzeug 7 und das Verdichten und Formen des Wickels zu einem Tampon 2 (Figur 7) umfasst. Zum Abstreifen des Wickels 10 ist ein Abstreifwerkzeug 11 vorgesehen, das längs verschiebbar auf dem Haltewerkzeug 7 sitzt und einen Längsschlitz 16 aufweist, damit später das Kabel 4 entfernt werden kann. Durch eine relative Längsbewegung des Abstreifwerkzeugs 11 gegenüber dem Haltewerkzeug 7 wird der Wickel 10 vom Haltewerkzeug 7 abgestreift und in ein Formwerkzeug 12 geschoben, das eine Öffnung mit einem Innendurchmesser aufweist, der kleiner ist als der Aussendurchmesser des Wickels 10 und im Wesentlichen dem Durchmesser des fertigen Tampons 2 (siehe Figur 7) entspricht. Durch die genannte Durchmesserdifferenz wird der Wickel radial komprimiert. Das Ende der Öffnung im Formwerkzeug 12 ist gerundet und hat vorzugsweise die Form einer Halbkugel. Durch das Abstreifwerkzeug 11 wird der Wickel 10 auch axial verdichtet, so dass der fertige Tampon an seinem Einführungsende einen gerundeten Kopf 3 (Figur 7) erhält. Das Formwerkzeug 12 ist geheizt, beispielsweise auf 100 °C und der komprimierte Wickel 10 verbleibt für eine gewisse Zeit, beispielsweise 20 Sekunden im Formwerkzeug 12. Dies führt dazu, dass der fertige Tampon nach dem Entfernen aus dem Formwerkzeug 12 formstabil bleibt. Auch kann die Hitze zum teilweisen Verschweissen des Vliesbandes 9 dienen, das zu diesem Zweck vorzugsweise aus einem schmelzbaren Material besteht. Zum Entfernen wird der Tampon 2 mittels eines Ausstossers 13 aus dem Formwerkzeug 12 geschoben.

Figur 7 zeigt die fertige Tamponanordnung 1 mit dem Tampon 2, dem gerundeten Kopf 3, dem Kabel 4 und der Energieversorgungs- und Steuereinheit 5.

Die Figuren 8 und 9 zeigen, dass die Kapsel 6 auch von Faserband 8 umwickelt werden kann, ohne dass sie vorher im Haltewerkzeug 7 gewendet wird. Dazu werden die Kapsel 6 und das Haltewerkzeug 7 zunächst mit parallelen Längsachsen unter Zwischenlage des Faserbandes 8 nebeneinander angeordnet. Dann wird die Kapsel 6 und/oder das Haltewerkzeug 7 parallel verschoben, so dass sich schliesslich die Kapsel 6 wie in Figur 8 gezeigt zwischen den Schenkeln des Haltewerkzeugs 7 befindet, wobei sich zwischen ihr und jedem Schenkel des Haltewerkzeuges 7 eine Lage des Faserbandes 8 befindet. Figur 9 zeigt eine Draufsicht auf die Kapsel 6 und das Haltewerkzeug 7 ausgehend von der Darstellung in Figur 8, nach abgeschlossenem Wickelvorgang.

Eine weitere Möglichkeit, die Kapsel 6, das Haltewerkzeug 7 und das Faserband 8 in eine Ausgangslage für das Wickeln zu bringen, ist in Figur 10 dargestellt. Hierbei wird vorzugsweise zuerst das Faserband 8 wie dargestellt zwischen die Schenkel des Haltewerkzeuges 7 gebracht und anschliessend wird die Kapsel 6 seitwärts in das Haltewerkzeug 7 eingeschoben, derart, dass sie auf der in der Figur unteren Seite direkt mit einem Schenkel des Haltewerkzeuges 7 in Kontakt steht und auf der in der Figur oberen Seite zwischen der Kapsel 6 und dem oberen Schenkel des Haltewerkzeugs 7 eine Lage des Faserbandes 8 liegt.

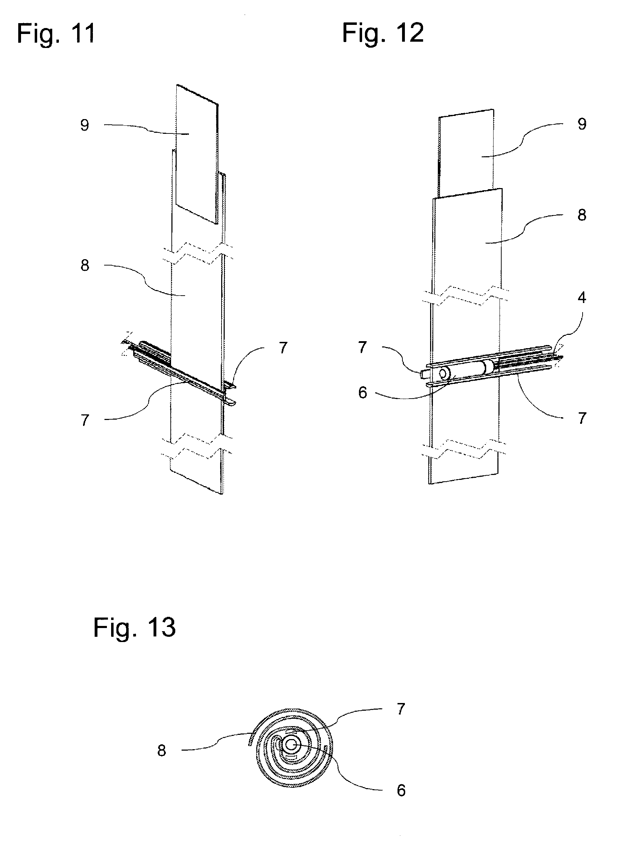

Schliesslich zeigen die Figuren 11 bis 13 noch eine andere Möglichkeit, die Kapsel 6, das Haltewerkzeug 7 und das Faserband 8 in eine Ausgangslage für das Wickeln zu bringen. Im Unterschied zu den vorangehend beschriebenen Verfahren hat das Haltewerkzeug 7 in diesem Fall drei Gabelschenkel. Dadurch wird es möglich, die Kapsel 6 und das Faserband in beliebiger Reihenfolge zwischen die Gabelschenkel zu bringen. Beispielsweise kann wie in Figur 11 dargestellt das Faserband 8 zwischen den links in dieser Figur dargestellten Schenkel und die beiden anderen Schenkel des Haltewerkzeuges 7 eingelegt werden und die Kapsel 6 wird zwischen die beiden anderen Schenkel eingeschoben, wie dies in Figur 12 zu sehen ist. Figur 13 zeigt in einer Stirnansicht den Verlauf des Faserbandes 8 in Bezug auf die Kapsel 6 und das Haltewerkzeug 7 nach dem Wickeln.

**Bezugszeichenliste** 1 Tamponanordnung 2 Tampon 3 Kopf 4 Kabel 5 Energieversorgungs- und Steuereinheit 6 Vibrationsantrieb 7 Haltewerkzeug 8 Faserband 9 Vliesband 10 Wickel 11 Abstreifwerkzeug 12 Formwerkzeug 13 Ausstosser 14 Aussenseite 15 vormontierte Anordnung 16 Längsschlitz 17 18 19 20

## Patentansprüche

1. Verfahren zum Umhüllen eines Körpers (6) mit Fasermaterial, **dadurch gekennzeichnet, dass** Fasermaterial (8) um den Körper (6) herum gewickelt wird.

2. Verfahren nach Anspruch 1, **dadurch gekennzeichnet, dass** das Fasermaterial als Flächengebilde (8) ausgebildet ist.

3. Verfahren nach Anspruch 2, **dadurch gekennzeichnet, dass** das Fasermaterial als Band (8) ausgebildet ist.

4. Verfahren nach einem der vorangehenden Ansprüche, **dadurch gekennzeichnet, dass** der Körper (6) eine vorzugsweise längliche Kapsel mit einem Inhalt ist.

5. Verfahren nach Anspruch 4, **dadurch gekennzeichnet, dass** der Inhalt der Kapsel (6) einen Energieverbraucher umfasst.

6. Verfahren nach Anspruch 5, **dadurch gekennzeichnet, dass** der Energieverbraucher ein Vibrationsgenerator ist.

7. Verfahren nach einem der vorangehenden Ansprüche, **dadurch gekennzeichnet, dass** der Körper (6) mit einem flexiblen Zugmittel (4) verbunden ist, das vorzugsweise mindestens einen Leiter für Energie enthält.

8. Verfahren nach einem der vorangehenden Ansprüche, **dadurch gekennzeichnet, dass** der Körper (6) in einem Haltewerkzeug (7) gehalten wird und dass das Haltewerkzeug (7) mit dem darin gehaltenen Körper (6) und das Fasermaterial (8) relativ zueinander rotiert werden, um einen Wickel (10) zu bilden.

9. Verfahren nach Anspruch 8, **dadurch gekennzeichnet, dass** das Haltewerkzeug (7) gabelförmig ist und mindestens zwei Gabelschenkel aufweist.

10. Verfahren nach Anspruch 9, **dadurch gekennzeichnet, dass** das Fasermaterial vor dem Rotieren zwischen den Körper (6) und mindestens einen der Gabelschenkel gebracht wird.

11. Verfahren nach Anspruch 10, **dadurch gekennzeichnet, dass** das Fasermaterial vor dem Rotieren zwischen den Körper (6) und mindestens zwei der Gabelschenkel gebracht wird.

12. Verfahren nach den Ansprüchen 7 und 11, **dadurch gekennzeichnet, dass** das Fasermaterial (8) in einer Schleife um den Körper gelegt wird und dass das flexible Zugmittel (4) über die Schleife parallel zum Körper (6) geführt wird.

13. Verfahren nach Anspruch 12, **dadurch gekennzeichnet, dass** der Körper (6) in das Haltewerkzeug (7) eingesetzt wird, dass das Fasermaterial (8) neben dem Haltewerkzeug (7) axial neben dem Körper (6) im Bereich des Zugmittels (4) platziert wird und dass der Körper (6) um eine senkrecht zu seiner Längsachse verlaufende Achse gewendet wird, derart, dass der Körper (6) das Fasermaterial (8) unter Bildung der Schleife in das Haltewerkzeug zieht.

14. Verfahren nach einem der Ansprüche 8 bis 13, **dadurch gekennzeichnet, dass** nach dem Rotieren der entstandene Wickel (10) axial vom Haltewerkzeug (7) abgestreift und in ein Formwerkzeug (12) gebracht wird.

15. Verfahren nach Anspruch 14, **dadurch gekennzeichnet, dass** das Formwerkzeug (12) einen Innendurchmesser hat, der kleiner ist als der Aussendurchmesser des Wickels (10).

16. Verfahren nach einem der Ansprüche 14 bis 15, **dadurch gekennzeichnet, dass** dem Wickel mit dem Formwerkzeug (12) eine gerundete Spitze (3) gegeben wird.

17. Verfahren nach einem der Ansprüche 14 bis 16, **dadurch gekennzeichnet, dass** das Formwerkzeug (12) beheizt wird.
